# EUROPEAN PATENT SPECIFICATION

(11) **EP 0 810 289 B1**
(45) Date of publication and mention of the grant of the patent: **25.08.2004**
(21) Application number: 96201495.7
(22) Date of filing: 29.05.1996
(51) Int. Cl.: C12N 15/57, C12N 9/52, C12N 15/11, C12N 1/21, A23C 9/123, A23C 19/032

(54) **Starter strains expressing a protease of Lactobacillus bulgaricus**
Starterkulturen die eine Protease von Lactobacillus bulgaricus exprimieren
Ferment à base de bactéries exprimant une protéase de Lactobacillus bulgaricus

(43) Date of publication of application: 03.12.1997
(73) Proprietor: SOCIETE DES PRODUITS NESTLE S.A., 1800 Vevey (CH)
(72) Inventor: Mollet, Beat, 1074 Mollie-Margot (CH); Germond, Jacques Edouard, 1023 Crissier (CH); Lapierre, Luciane, 1616 Attalens (CH)
(74) Representative: Becker Kurig Straus

(56) References cited:
- EP-A- 0 411 715
- MOLECULAR AND GENERAL GENETICS, vol. 248, 1995, pages 407-416, XP000608185 J-E GERMOND ET AL: "A new mobile genetic element in Lactobacillus delbrueckii subsp. bulgaricus"
- APPLIED MICROBIOLOGY ANS BIOTECHNOLOGY, vol. 36, 1991, pages 196-204, XP000608173 R. LALOI ET AL: "Cell-wall-associated proteinase of Lactobacillus delbreuckii subsp. bulgaricus CNRZ 397: differential extraction, purifucation and properties of the enzyme"
- JOURNAL OF APPLIED BACTERIOLOGY, vol. 41, 1976, pages 175-184, XP000608209 P. ARGYLE ET AL: "Production of cell-bound proteinase by Lactobacillus bulgaricus and its location in the bacterial cell "
- JOURNAL OF GENERAL MICROBIOLOGY, vol. 138, 1992, pages 1353-1364, XP000608160 A. HOLCK AND H. NAES: "Cloning, sequencing and expression of the gene encoding the cell-envelope-associated proteinase from Lactobacillus pparacasei subsp. paracasei NCDO 151"
- JOURNAL OF DAIRY RESEARCH , vol. 56, 1989, pages 285-296, XP000607001 A. VAFOPOULOU ET AL: "Accelerated ripening of Feta cheese, with heat-shocked cultures or microbial proteinases"
- APPLIED AND ENVIRONMENTAL MICROBIOLOGY, vol. 54, no. 1, January 1988, pages 231-238, XP000608155 J. KOK ET AL: "Nucleotide sequence of the cell wall proteinase gene of Streptococcus cremoris Wg2"
- JOURNAL OF GENERAL MICROBIOLOGY, vol. 138, 1992, pages 313-318, XP000608159 H. NAES AND J NISSEN-MEYER: "Purification and N-terminal amino acid sequence determination of the cell-wall-bound proteinase from Lactobacillus paracasei subsp. paracasei"

### SEQUENCE LISTING

(1) GENERAL INFORMATION:
   (i) APPLICANT:
      (A) NAME: SOCIETE DES PRODUITS NESTLE
      (B) STREET: AVENUE NESTLE 55
      (C) CITY: VEVEY
      (D) STATE: CANTON DE VAUD
      (E) COUNTRY: SWITZERLAND
      (F) POSTAL CODE (ZIP): 1800
      (G) TELEPHONE: (41).21.924.47.60
      (H) TELEFAX: (41).21.924.28.80
   (ii) TITLE OF INVENTION: Starter strains with accelerated fermentation properties
   (iii) NUMBER OF SEQUENCES: 2
   (iv) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.30 (EPO)
(2) INFORMATION FOR SEQ ID NO: 1:
   (i) SEQUENCE CHARACTERISTICS:
      (A) LENGTH: 7156 base pairs
      (B) TYPE: nucleic acid
      (C) STRANDEDNESS: double
      (D) TOPOLOGY: linear
   (ii) MOLECULE TYPE: DNA (genomic)
   (ix) FEATURE:
      (A) NAME/KEY: CDS
      (B) LOCATION:794..6631
   (ix) FEATURE:
      (A) NAME/KEY: misc_feature
      (B) LOCATION:608..627
      (D) OTHER INFORMATION:/function= "UP-element"
   (ix) FEATURE:
      (A) NAME/KEY: -35_signal
      (B) LOCATION:632..637
   (ix) FEATURE:
      (A) NAME/KEY: -10_signal
      (B) LOCATION:654..659
   (ix) FEATURE:
      (A) NAME/KEY: RBS
      (B) LOCATION:784..790
   (xi) SEQUENCE DESCRIPTION: SEQ ID NO: 1:
   TCGTGGGCTT TATTACCGGG CCAGTCGTTG ACAGCAAGTA TGACTACATC GAGGATTGGA    60
   TGTATGAAAA AGAGACAGAG AGTTTGGCAG GTCCCGGCGG CAACCAAATG GTCTTGACCA   120
   TCGCGGTTCA TCCGGATTAC CGGGGCCGGG GGATTGGCAG CCAGCTTTTA GATAGATTCG   180
   CGGAGACCGC TAAGGAAATG AAGCGGGGGC GGATCGCCTT GACCTGTCTG GAAGACCGGG   240
   TGCCATTTTA CGAAAAGAAT GGCTACGTCA ACCATGGGCA AGCTGAGTCA GAGCATGCCG   300
   GGGAAATCTG GTACAACATG ATCAAAGAGA TAGAAGGCTA GAAAGCGGTC AGCAGGTGCT   360
   GGCTGCTTTT TCTTTTGCTT TATAAGTGAA AAAATTTAAT GTGAATCTAA CTTAAAAAGC   420
   TATATCTAAA ATTTGGCAAT ATTCTTTTCT TAAATGAAAA AGCAGATGGA ACTATGTTTA   480
   AATAGGGACG GTGTTATTAT AATCAAAAAG GTAACACTAT ACCACGACTT TAGCGAATCA   540
   ACTTTGAAGT TAGTATTTTG TGGATTTTTA ACTTCATAGC ACGTTATTTT CTCGCGTATT   600
   GCTGAAATAA TGTGCTTTTT GTTTTTTGGC GTTCAGAAAT TACTTTTCCA CTGTTTGATT   660
   TAAAATTCTG CTAAAAAACA TTTAACTTTA ATTTAAACTA TGGTATGATT ATGAAATGTT      720
   AGTGAGCCGA AGCTGCGTGA TGAAAGGCTT GCTAATAAAA ATATTTATTT GTCTAAAGGA      780
   GTTAAGGAAA CAG ATG CAG AAG AAA AAA TCC GCA CGC CAT TTG AAC AAA         829
                  Met Gln Lys Lys Lys Ser Ala Arg His Leu Asn Lys
                   1               5                  10
   GTG GCT GAA TTA GCC GCA GCA CTG CTC CTA TCA GCG AGT CCA CTG GCG        877
   Val Ala Glu Leu Ala Ala Ala Leu Leu Leu Ser Ala Ser Pro Leu Ala
           15                  20                  25
   GGA ACT TTC CAG TCA GCC GCT TTT GTC CAA GCT GCC AGT CAA GAA ACG        925
   Gly Thr Phe Gln Ser Ala Ala Phe Val Gln Ala Ala Ser Gln Glu Thr
       30                  35                  40
   GTT AGT CCA CGG TCG GCT AGC CGG GCA GCA TTG ACT AAG TAC TTG CAG        973
   Val Ser Pro Arg Ser Ala Ser Arg Ala Ala Leu Thr Lys Tyr Leu Gln
   45                  50                  55                  60
   CAA GAG CAG CGC TAT AAC GCT AAG AAG AGT TAC AGC AAG TTC CAA GAG       1021
   Gln Glu Gln Arg Tyr Asn Ala Lys Lys Ser Tyr Ser Lys Phe Gln Glu
                   65                  70                  75
   GCT GCT AAA GAG CAA AGA CAG GCT TCT GGC CAA GCT GTC AGC AAG AAG       1069
   Ala Ala Lys Glu Gln Arg Gln Ala Ser Gly Gln Ala Val Ser Lys Lys
               80                  85                  90
   AAT GAG TCC TCG GTC CGG GTG ATT GTC AGT TTG AAC AAG TCA GCG GCA       1117
   Asn Glu Ser Ser Val Arg Val Ile Val Ser Leu Asn Lys Ser Ala Ala
           95                  100                 105
   TTT GAT CAC ACC AGT AAG CCG ACT GGA TCA GCT GCC TCG GTG AAA AAG       1165
   Phe Asp His Thr Ser Lys Pro Thr Gly Ser Ala Ala Ser Val Lys Lys
       110                 115                 120
   ATT GAG CAG GCA AGT GAC CAA GTC AAG GAT GGT CAA GAA AAG GTT ATC       1213
   Ile Glu Gln Ala Ser Asp Gln Val Lys Asp Gly Gln Glu Lys Val Ile
   125                 130                 135                 140
   AAG CAG GTA GAA GAA ATC ACC GGC AAC AAG GTC CGC CGG CAG TTC GGC       1261
   Lys Gln Val Glu Glu Ile Thr Gly Asn Lys Val Arg Arg Gln Phe Gly
                   145                 150                 155
   TAC TTG GTC AAT GCT TTT TCA ATC GAT ATG GAT TTA GAC GAT ATC GAC       1309
   Tyr Leu Val Asn Ala Phe Ser Ile Asp Met Asp Leu Asp Asp Ile Asp
               160                 165                 170
   AAG GTT AAG GAT CTG CCC CAG GTT AAG AAC GTT ACG CCT GTC AAG GTT       1357
   Lys Val Lys Asp Leu Pro Gln Val Lys Asn Val Thr Pro Val Lys Val
           175                 180                 185
   TAC CAC CCA ACC GAT GAG AGT GCT GAT CAA ATG GCC CAA GTG CAG GAC       1405
   Tyr His Pro Thr Asp Glu Ser Ala Asp Gln Met Ala Gln Val Gln Asp
       190                 195                 200
   GTT TGG CAA GAA CAA AAG CTC AAG GGT GAA GGT ATG GTC ATT TCG ATC       1453
   Val Trp Gln Glu Gln Lys Leu Lys Gly Glu Gly Met Val Ile Ser Ile
   205                 210                 215                 220
   ATT GAT ACA GGG ATC GAC TCC AGC CAC CAG GAC TTG AAG TTG GAC AGT       1501
   Ile Asp Thr Gly Ile Asp Ser Ser His Gln Asp Leu Lys Leu Asp Ser
                   225                 230                 235
   GGC GTC AGC ACC GCG TTG AGT AAG AGT GAG GTT GAA AGC GAC AAG AGT       1549
   Gly Val Ser Thr Ala Leu Ser Lys Ser Glu Val Glu Ser Asp Lys Ser
               240                 245                 250
   AAG CTA GGT CAT GGG AAG TAT TAC ACG GAG AAG GTT CCT TAC GGC TAT 1597
   Lys Leu Gly His Gly Lys Tyr Tyr Thr Glu Lys Val Pro Tyr Gly Tyr
   255 260 265
   AAC TAC GCT GAT AAG AAC GAC CAA ATC GTG GAC AAT GGG TGT GGC GAA 1645
   Asn Tyr Ala Asp Lys Asn Asp Gln Ile Val Asp Asn Gly Cys Gly Glu
   270 275 280
   ATG CAC GGC CAG CAC GTG GCC GGG ATT GCC GGG GCC AAT GGA CAA GTC 1693
   Met His Gly Gln His Val Ala Gly Ile Ala Gly Ala Asn Gly Gln Val
   285 290 295 300
   AAA GGT GTT GCT CCT GAT GCC CAG CTT TTG GCA ATG AAG GTC TTC TCC 1741
   Lys Gly Val Ala Pro Asp Ala Gln Leu Leu Ala Met Lys Val Phe Ser
   305 310 315
   AAC AAT GCC AAG AAT TCT GGA GCC TAT GAT GAC GAC ATC ATT TCC GCG 1789
   Asn Asn Ala Lys Asn Ser Gly Ala Tyr Asp Asp Asp Ile Ile Ser Ala
   320 325 330
   ATC GAA GAT TCA GTT AAG CTG GGG GCC GAC GTT ATC AAC ATG AGT TTG 1837
   Ile Glu Asp Ser Val Lys Leu Gly Ala Asp Val Ile Asn Met Ser Leu
   335 340 345
   GGC TCA GTT TCA TCA GAT GTC GGT CCA AGT GAT CCA CAG CAA CAA GCC 1885
   Gly Ser Val Ser Ser Asp Val Gly Pro Ser Asp Pro Gln Gln Gln Ala
   350 355 360
   GTA GCA AAG GCA TCA GAG GCT GGC GTC ATT AAT GTT ATC TCT GCT GGG 1933
   Val Ala Lys Ala Ser Glu Ala Gly Val Ile Asn Val Ile Ser Ala Gly
   365 370 375 380
   AAC TCC GGT GTT GCC GGG TCG ACT GCG GAT GGC AAT CCA GTC AAT AAT 1981
   Asn Ser Gly Val Ala Gly Ser Thr Ala Asp Gly Asn Pro Val Asn Asn
   385 390 395
   ACC GGG ACA AGT GAA CTC AGT ACT GTT GGC ACG CCA GGG GTT ACA CCG 2029
   Thr Gly Thr Ser Glu Leu Ser Thr Val Gly Thr Pro Gly Val Thr Pro
   400 405 410
   GAT GCT CTG ACG GTT GCC TCT GCT GAA AAC AGC AAG GTG ACC ACC GAC 2077
   Asp Ala Leu Thr Val Ala Ser Ala Glu Asn Ser Lys Val Thr Thr Asp
   415 420 425
   ACA GTT AAA GAC GAA CTC GGT GGG GTC ACC TTT AGC TCA AAT TCA GAA 2125
   Thr Val Lys Asp Glu Leu Gly Gly Val Thr Phe Ser Ser Asn Ser Glu
   430 435 440
   TTG AAG GGT GCT GCA CAA GTA ACA ACC CAG CTG GAA AGC AAC TAT TCC 2173
   Leu Lys Gly Ala Ala Gln Val Thr Thr Gln Leu Glu Ser Asn Tyr Ser
   445 450 455 460
   GTT TTA ACC AAG AAG CTT AAG CTT GTT GAC ATG GGC TTG GGC GGT GCA 2221
   Val Leu Thr Lys Lys Leu Lys Leu Val Asp Met Gly Leu Gly Gly Ala
   465 470 475
   GAT GAC TAT ACT GCT GAA AAG AAG GCT GAG GTT AAG GGA CAA CTT GCA 2269
   Asp Asp Tyr Thr Ala Glu Lys Lys Ala Glu Val Lys Gly Gln Leu Ala
   480 485 490
   GTT GTT AAA CGG GGA GCT TAT ACC TTC TCA GCG AAA GTT GCC AAT GCC 2317
   Val Val Lys Arg Gly Ala Tyr Thr Phe Ser Ala Lys Val Ala Asn Ala
   495 500 505
   AAG GCA GCT GGC GCG GCA GGG ATC GTC ATT TAC AAT AGC GAG GAT GAC 2365
   Lys Ala Ala Gly Ala Ala Gly Ile Val Ile Tyr Asn Ser Glu Asp Asp
   510 515 520
   GGC TTA CTT TCA ATG TCA CTG GAC GAC AAG ACT TTC CCA ACC TTG GGG 2413
   Gly Leu Leu Ser Met Ser Leu Asp Asp Lys Thr Phe Pro Thr Leu Gly
   525 530 535 540
   ATG AGT AAG GCT GAT GGG AAG TTC TGG CTA AAG CAG CAA AAG AAG GTA 2461
   Met Ser Lys Ala Asp Gly Lys Phe Trp Leu Lys Gln Gln Lys Lys Val
   545 550 555
   AGA GCA TCA AGG CTA AAA TTC GGT ACT GCT TTA ATC GAT AAC TCC AGG 2509
   Arg Ala Ser Arg Leu Lys Phe Gly Thr Ala Leu Ile Asp Asn Ser Arg
   560 565 570
   GCG GGA AAG ATG AGT GAC TTT ACC AGC TGG GGA CCA ACT CCT GAA TTA 2557
   Ala Gly Lys Met Ser Asp Phe Thr Ser Trp Gly Pro Thr Pro Glu Leu
   575 580 585
   GAC TTC AAG CCA GAA ATC ACT GCT CCA GGC GGG AAG ATC TAC TCG CTG 2605
   Asp Phe Lys Pro Glu Ile Thr Ala Pro Gly Gly Lys Ile Tyr Ser Leu
   590 595 600
   GCC AAC GAC AAC AAG TAC CAG CAA ATG AGT GGG ACT TCA ATG GCT TCA 2653
   Ala Asn Asp Asn Lys Tyr Gln Gln Met Ser Gly Thr Ser Met Ala Ser
   605 610 615 620
   CCA TTT GTC GCA GGT TCC GAA GCA TTA ATT TTG CAA GGT ATC AAG AAA 2701
   Pro Phe Val Ala Gly Ser Glu Ala Leu Ile Leu Gln Gly Ile Lys Lys
   625 630 635
   CAA GGT TTG AAC TTG TCC GGG GAA GAA TTA GTA CAA TTT GCT AAG AAT 2749
   Gln Gly Leu Asn Leu Ser Gly Glu Glu Leu Val Gln Phe Ala Lys Asn
   640 645 650
   TCA GCC ATG AAC ACT TCG CAC CCC GTC TAT GAC ACT GAG CAT ACC AAG 2797
   Ser Ala Met Asn Thr Ser His Pro Val Tyr Asp Thr Glu His Thr Lys
   655 660 665
   GAA ATT ATT TCT CCC CGC CGG CAA GGG AGT GGC GAA ATC AAT GTT AAA 2845
   Glu Ile Ile Ser Pro Arg Arg Gln Gly Ser Gly Glu Ile Asn Val Lys
   670 675 680
   GAT GCC ATC AAC AAT ACG GTT GAG GTT AAG GCA GCC AAC GGC AAC GGG 2893
   Asp Ala Ile Asn Asn Thr Val Glu Val Lys Ala Ala Asn Gly Asn Gly
   685 690 695 700
   GCA GCA GCC CTG AAA GAA ATT GGC CGG CAG ACT ACT TTT AAG GTT ACC 2941
   Ala Ala Ala Leu Lys Glu Ile Gly Arg Gln Thr Thr Phe Lys Val Thr
   705 710 715
   TTG ACC AAT CAT GGT AAG AAG GCC CAG ACC TAT GCA GTT GAT AAT TAT 2989
   Leu Thr Asn His Gly Lys Lys Ala Gln Thr Tyr Ala Val Asp Asn Tyr
   720 725 730
   GGC GGT CCA TAC ACC CAG GCA ACC GAA GCG AAG AGT GGC GAA ATC TAT 3037
   Gly Gly Pro Tyr Thr Gln Ala Thr Glu Ala Lys Ser Gly Glu Ile Tyr
   735 740 745
   GAC ACA AAG ATT GTT AAG GGA CAG CTG ACG ACG GAG ACG CCA AAG GTA 3085
   Asp Thr Lys Ile Val Lys Gly Gln Leu Thr Thr Glu Thr Pro Lys Val
   750 755 760
   ACT GTC CAG CCT GGC GAA AGT GTG GAT GTA AGC TTT ACC TTG ACC TTG 3133
   Thr Val Gln Pro Gly Glu Ser Val Asp Val Ser Phe Thr Leu Thr Leu
   765 770 775 780
   CCA TAC AGT TTC CAG CGG CAA AAT TTT GTT GAA GGC TAT GTT GGC TTT 3181
   Pro Tyr Ser Phe Gln Arg Gln Asn Phe Val Glu Gly Tyr Val Gly Phe
   785 790 795
   GAA GCA AAA GAC CAG GCA ACA CCA AAC CTG GTT CTT CCA TAC ATG GGC      3229
   Glu Ala Lys Asp Gln Ala Thr Pro Asn Leu Val Leu Pro Tyr Met Gly
               800                 805                 810
   TTT TTC GGC AGC TAC AGT CAA GCG AGT GTC AGC GCA CCA ATG CTT TAT      3277
   Phe Phe Gly Ser Tyr Ser Gln Ala Ser Val Ser Ala Pro Met Leu Tyr
           815                 820                 825
   GAA GGT GGC AAT TCA AAT CTA ATC AAC ACA ATT CAC TCC CTG GTT GGC      3325
   Glu Gly Gly Asn Ser Asn Leu Ile Asn Thr Ile His Ser Leu Val Gly
       830                 835                 840
   GTC ATG TTT TCT AAC AAC AAT GAT ATG CTC GGT CAC ACT GGC TAT GAG      3373
   Val Met Phe Ser Asn Asn Asn Asp Met Leu Gly His Thr Gly Tyr Glu
   845                 850                 855                 860
   GGT GAT GAT TAC AGC AAG TAT ACT GAT CCT GAT CTG ATC GCG ATT TCA      3421
   Gly Asp Asp Tyr Ser Lys Tyr Thr Asp Pro Asp Leu Ile Ala Ile Ser
                   865                 870                 875
   CCA AAT GGC GAT GGT AGC CGG GAC TAC GCA TAT CCT GTT CTC TTC TTT      3469
   Pro Asn Gly Asp Gly Ser Arg Asp Tyr Ala Tyr Pro Val Leu Phe Phe
               880                 885                 890
   GAC CGG AAC TAC AAG GAA TAC ACG GAA ACG ATT ACT GAT GCT CAA GGA      3517
   Asp Arg Asn Tyr Lys Glu Tyr Thr Glu Thr Ile Thr Asp Ala Gln Gly
           895                 900                 905
   AAC AAA GTT AAG AGC TTG GGC GTT GGC AAA GAG GGA ACT AAA GAT TAC      3565
   Asn Lys Val Lys Ser Leu Gly Val Gly Lys Glu Gly Thr Lys Asp Tyr
       910                 915                 920
   TAC AGT TCT TCA AGC GGT GAG TGG ACA ACC CAC TCA CTG GAC AAG TGG      3613
   Tyr Ser Ser Ser Ser Gly Glu Trp Thr Thr His Ser Leu Asp Lys Trp
   925                 930                 935                 940
   GAT GGC ACT GAC GCT GAC GGC CAA GTC GTC AAG GAT GGG CAA TAC ATC      3661
   Asp Gly Thr Asp Ala Asp Gly Gln Val Val Lys Asp Gly Gln Tyr Ile
                   945                 950                 955
   TAC AAG GTT GAA TTT ACC CCA GCA ATT GGC GGC CAA GAG CAG GAA TTG      3709
   Tyr Lys Val Glu Phe Thr Pro Ala Ile Gly Gly Gln Glu Gln Glu Leu
               960                 965                 970
   AAC ATC CCT GTC AAG GTT GAT AGC CAA GCG CCA GAA GTG AGT GAC TTG      3757
   Asn Ile Pro Val Lys Val Asp Ser Gln Ala Pro Glu Val Ser Asp Leu
           975                 980                 985
   CAG GTT ACC AAA GAT GGC AAG CTC CGT TTG AAG GCT AAG GAT TCC GGC      3805
   Gln Val Thr Lys Asp Gly Lys Leu Arg Leu Lys Ala Lys Asp Ser Gly
       990                 995                 1000
   AGC GGT CTT GAC ATG ACA ATG TTT GTT GCG GCA GTA AAC GGT GAA GAG      3853
   Ser Gly Leu Asp Met Thr Met Phe Val Ala Ala Val Asn Gly Glu Glu
   1005                1010                1015                1020
   CAG AAG CTG GCG CTG GCT CCG GTT AAG GGA GAA AGT AAT GTT TAC GAG      3901
   Gln Lys Leu Ala Leu Ala Pro Val Lys Gly Glu Ser Asn Val Tyr Glu
                   1025                1030                1035
   TCA ACG TCT GCC TTG ACT GGA CTT AAG GAC GGC AAG AAC CAA GTA GAA      3949
   Ser Thr Ser Ala Leu Thr Gly Leu Lys Asp Gly Lys Asn Gln Val Glu
               1040                1045                1050
   ACT GTT TTG GCT GAC TAT GCC GGC AAT GTT GGC TAT GCT GCA ACT TTC      3997
   Thr Val Leu Ala Asp Tyr Ala Gly Asn Val Gly Tyr Ala Ala Thr Phe
           1055                1060                1065
   TCC AGT CAA AAT AAC GAT GCT GAT AAC AAG CTT CTT CTC TTT AAC TTA      4045
   Ser Ser Gln Asn Asn Asp Ala Asp Asn Lys Leu Leu Leu Phe Asn Leu
       1070                1075                1080
   GCT GAC GGT CAA AAG ATC ACT AGT CAA AGC CCG GCT TAC GAT CAA GAG      4093
   Ala Asp Gly Gln Lys Ile Thr Ser Gln Ser Pro Ala Tyr Asp Gln Glu
   1085                1090                1095                1100
   AAA GAA ACT TAC ACT GTA ACT GGT ACT TAC AAG AAG AAT GCC AAG CTT      4141
   Lys Glu Thr Tyr Thr Val Thr Gly Thr Tyr Lys Lys Asn Ala Lys Leu
                   1105                1110                1115
   AAG TTT AAT GAT GTC GAG GCA GAA AGC GAT AAA AAT GGC TAC TTT GAA      4189
   Lys Phe Asn Asp Val Glu Ala Glu Ser Asp Lys Asn Gly Tyr Phe Glu
               1120                1125                1130
   GTT AAG CTG CCA GTC AAG GAT GGA CAA AAC CAG CTT TTG ATC AAG GAT      4237
   Val Lys Leu Pro Val Lys Asp Gly Gln Asn Gln Leu Leu Ile Lys Asp
           1135                1140                1145
   GGG GAC CAA ATC CTT GAA GCA GTC AAC TTC ACT GTT AAG GCT GAA GGC      4285
   Gly Asp Gln Ile Leu Glu Ala Val Asn Phe Thr Val Lys Ala Glu Gly
       1150                1155                1160
   CGA AAG TCA GCG TTG ACG AAG AAA GAT CAG GCC GCA TTT TGG CTA AGG      4333
   Arg Lys Ser Ala Leu Thr Lys Lys Asp Gln Ala Ala Phe Trp Leu Arg
   1165                1170                1175                1180
   ATG ATT CCT ATA CTT TAT CCG GGA CGG TAT CTG GCC TTG GCG CAA ACG      4381
   Met Ile Pro Ile Leu Tyr Pro Gly Arg Tyr Leu Ala Leu Ala Gln Thr
                   1185                1190                1195
   GGC AAA CTG GAA CTG ACA AAC TTG TCT GAC AAG AGC AAG ACT GAT AAG      4429
   Gly Lys Leu Glu Leu Thr Asn Leu Ser Asp Lys Ser Lys Thr Asp Lys
               1200                1205                1210
   TTA ACG GTT GAC CAG GAC GGC AAG TTC TCA CAA AAG GTT GAC TTG AAC      4477
   Leu Thr Val Asp Gln Asp Gly Lys Phe Ser Gln Lys Val Asp Leu Asn
           1215                1220                1225
   TAC GGT GAC AAT CCT TTT GAA CTG ACG GCA ACT GAT GCG GAT GGC AAT      4525
   Tyr Gly Asp Asn Pro Phe Glu Leu Thr Ala Thr Asp Ala Asp Gly Asn
       1230                1235                1240
   GTT ACA AAG AAG GAC GTA ACG ATT TTC ACC GCT AGA AGT TAC ACT TAC      4573
   Val Thr Lys Lys Asp Val Thr Ile Phe Thr Ala Arg Ser Tyr Thr Tyr
   1245                1250                1255                1260
   AAC AAG GAT ATG CTG ACT TTT GAC AAT ATC GCC AGC GAT CTG ACG GTT      4621
   Asn Lys Asp Met Leu Thr Phe Asp Asn Ile Ala Ser Asp Leu Thr Val
                   1265                1270                1275
   ATT GGC AAG ATG ACG CCG GGT TAC GAT GAG AAG GAC CAC AGC TTC ACA      4669
   Ile Gly Lys Met Thr Pro Gly Tyr Asp Glu Lys Asp His Ser Phe Thr
               1280                1285                1290
   GTA ACG GGT AAA CTC GCG TAC CCA GTC GCC CGC TTC CAG TTA ATC GGG      4717
   Val Thr Gly Lys Leu Ala Tyr Pro Val Ala Arg Phe Gln Leu Ile Gly
           1295                1300                1305
   GAT GAC GTT AAG TAC GAC CCA GAT ACA TTG AAG TTC AGC TAC ACG ATC      4765
   Asp Asp Val Lys Tyr Asp Pro Asp Thr Leu Lys Phe Ser Tyr Thr Ile
       1310                1315                1320
   AAA GAC TTA AAG AAC GGC AAC CAT TCA TTG ACT GCT CTG GTT CAA GAT      4813
   Lys Asp Leu Lys Asn Gly Asn His Ser Leu Thr Ala Leu Val Gln Asp
   1325                1330                1335                1340
   CCA CGG CTT AAT GAC GGC AAG CCA GTC GTC GAA TGG GGC TAC AAG CTC    4861
   Pro Arg Leu Asn Asp Gly Lys Pro Val Val Glu Trp Gly Tyr Lys Leu
                   1345                1350                1355
   TGG GTT GAC TTA GCA GCG CCA AGC CTG CAA TTG GAA GGG ATG TCC TTA    4909
   Trp Val Asp Leu Ala Ala Pro Ser Leu Gln Leu Glu Gly Met Ser Leu
               1360                1365                1370
   GGT GAG GAT GGC CAG TTG GCT GTC TAC ACC AAC AAG GAT GTT TAC GAT    4957
   Gly Glu Asp Gly Gln Leu Ala Val Tyr Thr Asn Lys Asp Val Tyr Asp
           1375                1380                1385
   CTT AAG GCA ACG ATC AAT GAC AAC TTG AGC GGA TAC AGC CTG CAA GTT    5005
   Leu Lys Ala Thr Ile Asn Asp Asn Leu Ser Gly Tyr Ser Leu Gln Val
       1390                1395                1400
   GGC AGC GAT ACA GCA TAC CAA GAC AAG ACT TAC AAG GTG TTT AAC GAA    5053
   Gly Ser Asp Thr Ala Tyr Gln Asp Lys Thr Tyr Lys Val Phe Asn Glu
   1405                1410                1415                1420
   GAC TTC TTT AAG AAT AGG GAT GCA GTC AAG GTC AGC TAC CCG ATC AAG    5101
   Asp Phe Phe Lys Asn Arg Asp Ala Val Lys Val Ser Tyr Pro Ile Lys
                   1425                1430                1435
   GCT GAG AAA GAC GGA TTC CGG AAG GTC AAG GTC ACT TTG ACT GAT GGT    5149
   Ala Glu Lys Asp Gly Phe Arg Lys Val Lys Val Thr Leu Thr Asp Gly
               1440                1445                1450
   AGT GAC AAC AAA ACT GAA CAA GAC TTT ACC CTG TAC AAC CAC CAG GCA    5197
   Ser Asp Asn Lys Thr Glu Gln Asp Phe Thr Leu Tyr Asn His Gln Ala
           1455                1460                1465
   GAT CTT GAG GCA CCG GAG GTT TCC GCT TCA GAA AGC AAG AAG ACC AAC    5245
   Asp Leu Glu Ala Pro Glu Val Ser Ala Ser Glu Ser Lys Lys Thr Asn
       1470                1475                1480
   CAA GCT GTT CAG CTG AAG GTC GGA AAT CTG TCC GAT GTA CAA AAG TCT    5293
   Gln Ala Val Gln Leu Lys Val Gly Asn Leu Ser Asp Val Gln Lys Ser
   1485                1490                1495                1500
   GCT GGC AAG TTT AAG GCG GCT GAT CTC TAC TAC TCA GTT GAC GGC AAG    5341
   Ala Gly Lys Phe Lys Ala Ala Asp Leu Tyr Tyr Ser Val Asp Gly Lys
                   1505                1510                1515
   TCT TGG ACC AAG CTG GAC AAG GAT ACT GTA CAG GTA GCC GAA AAC GGA    5389
   Ser Trp Thr Lys Leu Asp Lys Asp Thr Val Gln Val Ala Glu Asn Gly
               1520                1525                1530
   AAG GTT GAG TTC AAG TAC CAA GAC GTT TAC GGC AAT GAG TCA AAG GTT    5437
   Lys Val Glu Phe Lys Tyr Gln Asp Val Tyr Gly Asn Glu Ser Lys Val
           1535                1540                1545
   ACG ACT TAT GAA GTC AAG AAT ATC GTC AAG GAA GTC GCT GCC CAG CCG    5485
   Thr Thr Tyr Glu Val Lys Asn Ile Val Lys Glu Val Ala Ala Gln Pro
       1550                1555                1560
   GAA TTG AAG CTG ACT CCG GAT GGA GAA GGC AAG GTC AAG GCT GAG CTT    5533
   Glu Leu Lys Leu Thr Pro Asp Gly Glu Gly Lys Val Lys Ala Glu Leu
   1565                1570                1575                1580
   GCT TTT GAC AAG AAG GAT GTT GAC AAG GAC TTC AAC CAC ATC AAG TAC    5581
   Ala Phe Asp Lys Lys Asp Val Asp Lys Asp Phe Asn His Ile Lys Tyr
                   1585                1590                1595
   AGC CTT GAT GGT GGC AAG TCC TGG ACT GAC TAC AAG GAT GCC TTT ACG    5629
   Ser Leu Asp Gly Gly Lys Ser Trp Thr Asp Tyr Lys Asp Ala Phe Thr
               1600                1605                1610
   CTG ACC CAC AAC GGA ACT GTG GAA TTC AAG AGC TAT GAC GAC GCT GGC        5677
   Leu Thr His Asn Gly Thr Val Glu Phe Lys Ser Tyr Asp Asp Ala Gly
               1615                1620                1625
   AAT GAG AGC CAA GTC TAC ACC AGT GTG GTC AAG GTA GAA CGG AAG CTG        5725
   Asn Glu Ser Gln Val Tyr Thr Ser Val Val Lys Val Glu Arg Lys Leu
           1630                1635                1640
   CCC GCT CCA GAT TTG ACT GGC ACG GTT GAA GCT GAT AAG AGT GTT GAG        5773
   Pro Ala Pro Asp Leu Thr Gly Thr Val Glu Ala Asp Lys Ser Val Glu
   1645                1650                1655                1660
   GTT AAG GCT GGC AAC GTT GAA CAG GCT GAA AAG GAT GCT GAC GGA AAA        5821
   Val Lys Ala Gly Asn Val Glu Gln Ala Glu Lys Asp Ala Asp Gly Lys
                   1665                1670                1675
   GTA ACT TTG CTT TAC TCA ACT GAT GGT AAG GAT TGG TCA AAG GTT GAT        5869
   Val Thr Leu Leu Tyr Ser Thr Asp Gly Lys Asp Trp Ser Lys Val Asp
               1680                1685                1690
   GGC GAA GTT AAG CTG ACT GAC AGC GGC CGG GCT ATG TTC AAG TAC CGC        5917
   Gly Glu Val Lys Leu Thr Asp Ser Gly Arg Ala Met Phe Lys Tyr Arg
           1695                1700                1705
   GAC GGG GAT GAC AAC GAA TCT GCT GTT GTC GTT TAT GAA GTC AAG AAG        5965
   Asp Gly Asp Asp Asn Glu Ser Ala Val Val Val Tyr Glu Val Lys Lys
       1710                1715                1720
   GTT GTT GAA GAA AAG ACT GCC GAG TTT AAG CCG GAA AAC AAG GAC GAT        6013
   Val Val Glu Glu Lys Thr Ala Glu Phe Lys Pro Glu Asn Lys Asp Asp
   1725                1730                1735                1740
   AGC AAG TCA GAC AGC AAG AGT GAT TCT AAG TCT GAT AAC AAG GCA GAA        6061
   Ser Lys Ser Asp Ser Lys Ser Asp Ser Lys Ser Asp Asn Lys Ala Glu
                   1745                1750                1755
   ACC AAG AGT GAT GTG AAG ACT GAC ACT AAG AGT GAT GTC AAA TCG GAC        6109
   Thr Lys Ser Asp Val Lys Thr Asp Thr Lys Ser Asp Val Lys Ser Asp
               1760                1765                1770
   GGC AAT GGC GAA CGT AAG TCC GAT AAC AAG CCA TCT GCC AAG GAT GAT        6157
   Gly Asn Gly Glu Arg Lys Ser Asp Asn Lys Pro Ser Ala Lys Asp Asp
           1775                1780                1785
   AAA AAG ACT GAC AGC AAG ACG ACT TCA AAG AGT AGC GCG AAG AAG ACT        6205
   Lys Lys Thr Asp Ser Lys Thr Thr Ser Lys Ser Ser Ala Lys Lys Thr
       1790                1795                1800
   AGT GCT AAG AAG AAC AAG AAG GCT TCT AAG AAG GCA AGC AAG AAG ACA        6253
   Ser Ala Lys Lys Asn Lys Lys Ala Ser Lys Lys Ala Ser Lys Lys Thr
   1805                1810                1815                1820
   GTT AAG AAG ACT AAG ACT TAC AAG AAG GTC AAG TTG ACT AAG CTG ACA        6301
   Val Lys Lys Thr Lys Thr Tyr Lys Lys Val Lys Leu Thr Lys Leu Thr
                   1825                1830                1835
   AAA GTC TAC AAT AAG AAG GGC AAG GTA GTT GGC AAG CTT AAG AAG AAG        6349
   Lys Val Tyr Asn Lys Lys Gly Lys Val Val Gly Lys Leu Lys Lys Lys
               1840                1845                1850
   ACT AGC GTC AAG CTA TTA AGC AAG AAG CAA AAG CTT CAT GGA AAG TAC        6397
   Thr Ser Val Lys Leu Leu Ser Lys Lys Gln Lys Leu His Gly Lys Tyr
           1855                1860                1865
   TAC TAC CGG GTT GGC AAG AAC AGA TAT ATT CTG GCA AGC AGC CTG CCG        6445
   Tyr Tyr Arg Val Gly Lys Asn Arg Tyr Ile Leu Ala Ser Ser Leu Pro
       1870                1875                1880
   AAA AAG ACT AAG AAG GTT AAG CAA GTT CGC GCA AGG AAG AAT GCC AAG      6493
   Lys Lys Thr Lys Lys Val Lys Gln Val Arg Ala Arg Lys Asn Ala Lys
   1885                1890                1895                1900
   GTC TAT AAC AAG AAA GGG AAG GTA GTT GGC CAT CTG AAA AAG AAG CAG      6541
   Val Tyr Asn Lys Lys Gly Lys Val Val Gly His Leu Lys Lys Lys Gln
                   1905                1910                1915
   AAG GTT AAG CTT TTA AGC AAG AAA CAG AAG CTT CAT GGC AAG TAC TAC      6589
   Lys Val Lys Leu Leu Ser Lys Lys Gln Lys Leu His Gly Lys Tyr Tyr
               1920                1925                1930
   TAC CGA ATT GGC AAG AAC AGA TAT GTT AAT GCA AAT GTG CTG              6631
   Tyr Arg Ile Gly Lys Asn Arg Tyr Val Asn Ala Asn Val Leu
           1935                1940                1945
   TAATTAGAGG GAGACAGGTT CCGAAAGGAA CTGTCTTTCT CGTTATAGGC ATTGGAGAGA    6691
   GAACGGGTTG TTAGTTTGAT GATATAATTA TTTTGAAATC TGGAGATAAG GATCGGAACT    6751
   TAGGCTTTGT ACTTGTTGAT CCGGCAATCT ATTCAATTCA GCATAATATT GCAAACTTGA    6811
   AAATTGCAGG TGATTTTCCA AAACGGGGTA CCGAGCTCGA ATTCACTGGC CGTCGTTTTA    6871
   CAACGTCGTG ACTGGAAACC TGCGTTACCA ACTTATCGCC TTGCAGACAT CCTTACTGTC    6931
   CAGCCTGGCG AAAGTGTGGA TGTAAGCTTT ACCTTGACCT TGCCATACAG TTTCCAGCGG    6991
   CAAAATTTTG TTGAAGGCTA TGTTGGCTTT GAAGCAAAAG ACCAGGCAAA TGACCGGAAC    7051
   TACAAGGAAT ACACGGAAAC GATTACTGAT GCTCAAGGAA ACAAAGTTAA GAGCTTGGGC    7111
   GTTGGCAAAG AGGGAACTAA AGATTACTAC AGTTCTTCAA GCGGT                    7156
(2) INFORMATION FOR SEQ ID NO: 2:
   (i) SEQUENCE CHARACTERISTICS:
      (A) LENGTH: 1946 amino acids
      (B) TYPE: amino acid
      (D) TOPOLOGY: linear
   (ii) MOLECULE TYPE: protein
   (xi) SEQUENCE DESCRIPTION: SEQ ID NO: 2:
   Met Gln Lys Lys Lys Ser Ala Arg His Leu Asn Lys Val Ala Glu Leu
    1               5                  10                  15
   Ala Ala Ala Leu Leu Leu Ser Ala Ser Pro Leu Ala Gly Thr Phe Gln
               20                  25                  30
   Ser Ala Ala Phe Val Gln Ala Ala Ser Gln Glu Thr Val Ser Pro Arg
           35                  40                  45
   Ser Ala Ser Arg Ala Ala Leu Thr Lys Tyr Leu Gln Gln Glu Gln Arg
       50                  55                  60
   Tyr Asn Ala Lys Lys Ser Tyr Ser Lys Phe Gln Glu Ala Ala Lys Glu
   65                  70                  75                      80
   Gln Arg Gln Ala Ser Gly Gln Ala Val Ser Lys Lys Asn Glu Ser Ser
                   85                  90                  95
   Val Arg Val Ile Val Ser Leu Asn Lys Ser Ala Ala Phe Asp His Thr
               100                 105                 110
   Ser Lys Pro Thr Gly Ser Ala Ala Ser Val Lys Lys Ile Glu Gln Ala
           115                 120                 125
   Ser Asp Gln Val Lys Asp Gly Gln Glu Lys Val Ile Lys Gln Val Glu
       130                 135                 140
   Glu Ile Thr Gly Asn Lys Val Arg Arg Gln Phe Gly Tyr Leu Val Asn
   145                 150                 155                 160
   Ala Phe Ser Ile Asp Met Asp Leu Asp Asp Ile Asp Lys Val Lys Asp
                   165                 170                 175
   Leu Pro Gln Val Lys Asn Val Thr Pro Val Lys Val Tyr His Pro Thr
               180                 185                 190
   Asp Glu Ser Ala Asp Gln Met Ala Gln Val Gln Asp Val Trp Gln Glu
           195                 200                 205
   Gln Lys Leu Lys Gly Glu Gly Met Val Ile Ser Ile Ile Asp Thr Gly
       210                 215                 220
   Ile Asp Ser Ser His Gln Asp Leu Lys Leu Asp Ser Gly Val Ser Thr
   225                 230                 235                 240
   Ala Leu Ser Lys Ser Glu Val Glu Ser Asp Lys Ser Lys Leu Gly His
                   245                 250                 255
   Gly Lys Tyr Tyr Thr Glu Lys Val Pro Tyr Gly Tyr Asn Tyr Ala Asp
               260                 265                 270
   Lys Asn Asp Gln Ile Val Asp Asn Gly Cys Gly Glu Met His Gly Gln
           275                 280                 285
   His Val Ala Gly Ile Ala Gly Ala Asn Gly Gln Val Lys Gly Val Ala
       290                 295                 300
   Pro Asp Ala Gln Leu Leu Ala Met Lys Val Phe Ser Asn Asn Ala Lys
   305                 310                 315                 320
   Asn Ser Gly Ala Tyr Asp Asp Asp Ile Ile Ser Ala Ile Glu Asp Ser
                   325                 330                 335
   Val Lys Leu Gly Ala Asp Val Ile Asn Met Ser Leu Gly Ser Val Ser
               340                 345                 350
   Ser Asp Val Gly Pro Ser Asp Pro Gln Gln Gln Ala Val Ala Lys Ala
           355                 360                 365
   Ser Glu Ala Gly Val Ile Asn Val Ile Ser Ala Gly Asn Ser Gly Val
       370                 375                 380
   Ala Gly Ser Thr Ala Asp Gly Asn Pro Val Asn Asn Thr Gly Thr Ser
   385                 390                 395                 400
   Glu Leu Ser Thr Val Gly Thr Pro Gly Val Thr Pro Asp Ala Leu Thr
                   405                 410                 415
   Val Ala Ser Ala Glu Asn Ser Lys Val Thr Thr Asp Thr Val Lys Asp
               420                 425                 430
   Glu Leu Gly Gly Val Thr Phe Ser Ser Asn Ser Glu Leu Lys Gly Ala
           435                 440                 445
   Ala Gln Val Thr Thr Gln Leu Glu Ser Asn Tyr Ser Val Leu Thr Lys
       450                 455                 460
   Lys Leu Lys Leu Val Asp Met Gly Leu Gly Gly Ala Asp Asp Tyr Thr
   465                 470                 475                 480
   Ala Glu Lys Lys Ala Glu Val Lys Gly Gln Leu Ala Val Val Lys Arg
                   485                 490                 495
   Gly Ala Tyr Thr Phe Ser Ala Lys Val Ala Asn Ala Lys Ala Ala Gly
               500                 505                 510
   Ala Ala Gly Ile Val Ile Tyr Asn Ser Glu Asp Asp Gly Leu Leu Ser
           515                 520                 525
   Met Ser Leu Asp Asp Lys Thr Phe Pro Thr Leu Gly Met Ser Lys Ala
       530                 535                 540
   Asp Gly Lys Phe Trp Leu Lys Gln Gln Lys Lys Val Arg Ala Ser Arg
   545                 550                 555                 560
   Leu Lys Phe Gly Thr Ala Leu Ile Asp Asn Ser Arg Ala Gly Lys Met
                   565                 570                 575
   Ser Asp Phe Thr Ser Trp Gly Pro Thr Pro Glu Leu Asp Phe Lys Pro
               580                 585                 590
   Glu Ile Thr Ala Pro Gly Gly Lys Ile Tyr Ser Leu Ala Asn Asp Asn
           595                 600                 605
   Lys Tyr Gln Gln Met Ser Gly Thr Ser Met Ala Ser Pro Phe Val Ala
       610                 615                 620
   Gly Ser Glu Ala Leu Ile Leu Gln Gly Ile Lys Lys Gln Gly Leu Asn
   625                 630                 635                 640
   Leu Ser Gly Glu Glu Leu Val Gln Phe Ala Lys Asn Ser Ala Met Asn
                   645                 650                 655
   Thr Ser His Pro Val Tyr Asp Thr Glu His Thr Lys Glu Ile Ile Ser
               660                 665                 670
   Pro Arg Arg Gln Gly Ser Gly Glu Ile Asn Val Lys Asp Ala Ile Asn
           675                 680                 685
   Asn Thr Val Glu Val Lys Ala Ala Asn Gly Asn Gly Ala Ala Ala Leu
       690                 695                 700
   Lys Glu Ile Gly Arg Gln Thr Thr Phe Lys Val Thr Leu Thr Asn His
   705                 710                 715                 720
   Gly Lys Lys Ala Gln Thr Tyr Ala Val Asp Asn Tyr Gly Gly Pro Tyr
                   725                 730                 735
   Thr Gln Ala Thr Glu Ala Lys Ser Gly Glu Ile Tyr Asp Thr Lys Ile
               740                 745                 750
   Val Lys Gly Gln Leu Thr Thr Glu Thr Pro Lys Val Thr Val Gln Pro
           755                 760                 765
   Gly Glu Ser Val Asp Val Ser Phe Thr Leu Thr Leu Pro Tyr Ser Phe
       770                 775                 780
   Gln Arg Gln Asn Phe Val Glu Gly Tyr Val Gly Phe Glu Ala Lys Asp
   785                 790                 795                 800
   Gln Ala Thr Pro Asn Leu Val Leu Pro Tyr Met Gly Phe Phe Gly Ser
                   805                 810                 815
   Tyr Ser Gln Ala Ser Val Ser Ala Pro Met Leu Tyr Glu Gly Gly Asn
               820                 825                 830
   Ser Asn Leu Ile Asn Thr Ile His Ser Leu Val Gly Val Met Phe Ser
           835                 840                 845
   Asn Asn Asn Asp Met Leu Gly His Thr Gly Tyr Glu Gly Asp Asp Tyr
       850                 855                 860
   Ser Lys Tyr Thr Asp Pro Asp Leu Ile Ala Ile Ser Pro Asn Gly Asp
   865 870 875 880
   Gly Ser Arg Asp Tyr Ala Tyr Pro Val Leu Phe Phe Asp Arg Asn Tyr
   885 890 895
   Lys Glu Tyr Thr Glu Thr Ile Thr Asp Ala Gln Gly Asn Lys Val Lys
   900 905 910
   Ser Leu Gly Val Gly Lys Glu Gly Thr Lys Asp Tyr Tyr Ser Ser Ser
   915 920 925
   Ser Gly Glu Trp Thr Thr His Ser Leu Asp Lys Trp Asp Gly Thr Asp
   930 935 940
   Ala Asp Gly Gln Val Val Lys Asp Gly Gln Tyr Ile Tyr Lys Val Glu
   945 950 955 960
   Phe Thr Pro Ala Ile Gly Gly Gln Glu Gln Glu Leu Asn Ile Pro Val
   965 970 975
   Lys Val Asp Ser Gln Ala Pro Glu Val Ser Asp Leu Gln Val Thr Lys
   980 985 990
   Asp Gly Lys Leu Arg Leu Lys Ala Lys Asp Ser Gly Ser Gly Leu Asp
   995 1000 1005
   Met Thr Met Phe Val Ala Ala Val Asn Gly Glu Glu Gln Lys Leu Ala
   1010 1015 1020
   Leu Ala Pro Val Lys Gly Glu Ser Asn Val Tyr Glu Ser Thr Ser Ala
   1025 1030 1035 1040
   Leu Thr Gly Leu Lys Asp Gly Lys Asn Gln Val Glu Thr Val Leu Ala
   1045 1050 1055
   Asp Tyr Ala Gly Asn Val Gly Tyr Ala Ala Thr Phe Ser Ser Gln Asn
   1060 1065 1070
   Asn Asp Ala Asp Asn Lys Leu Leu Leu Phe Asn Leu Ala Asp Gly Gln
   1075 1080 1085
   Lys Ile Thr Ser Gln Ser Pro Ala Tyr Asp Gln Glu Lys Glu Thr Tyr
   1090 1095 1100
   Thr Val Thr Gly Thr Tyr Lys Lys Asn Ala Lys Leu Lys Phe Asn Asp
   1105 1110 1115 1120
   Val Glu Ala Glu Ser Asp Lys Asn Gly Tyr Phe Glu Val Lys Leu Pro
   1125 1130 1135
   Val Lys Asp Gly Gln Asn Gln Leu Leu Ile Lys Asp Gly Asp Gln Ile
   1140 1145 1150
   Leu Glu Ala Val Asn Phe Thr Val Lys Ala Glu Gly Arg Lys Ser Ala
   1155 1160 1165
   Leu Thr Lys Lys Asp Gln Ala Ala Phe Trp Leu Arg Met Ile Pro Ile
   1170 1175 1180
   Leu Tyr Pro Gly Arg Tyr Leu Ala Leu Ala Gln Thr Gly Lys Leu Glu
   1185 1190 1195 1200
   Leu Thr Asn Leu Ser Asp Lys Ser Lys Thr Asp Lys Leu Thr Val Asp
   1205 1210 1215
   Gln Asp Gly Lys Phe Ser Gln Lys Val Asp Leu Asn Tyr Gly Asp Asn
   1220 1225 1230
   Pro Phe Glu Leu Thr Ala Thr Asp Ala Asp Gly Asn Val Thr Lys Lys
           1235                1240                1245
   Asp Val Thr Ile Phe Thr Ala Arg Ser Tyr Thr Tyr Asn Lys Asp Met
       1250                1255                1260
   Leu Thr Phe Asp Asn Ile Ala Ser Asp Leu Thr Val Ile Gly Lys Met
   1265                1270                1275                1280
   Thr Pro Gly Tyr Asp Glu Lys Asp His Ser Phe Thr Val Thr Gly Lys
                   1285                1290                1295
   Leu Ala Tyr Pro Val Ala Arg Phe Gln Leu Ile Gly Asp Asp Val Lys
               1300                1305                1310
   Tyr Asp Pro Asp Thr Leu Lys Phe Ser Tyr Thr Ile Lys Asp Leu Lys
           1315                1320                1325
   Asn Gly Asn His Ser Leu Thr Ala Leu Val Gln Asp Pro Arg Leu Asn
       1330                1335                1340
   Asp Gly Lys Pro Val Val Glu Trp Gly Tyr Lys Leu Trp Val Asp Leu
   1345                1350                1355                1360
   Ala Ala Pro Ser Leu Gln Leu Glu Gly Met Ser Leu Gly Glu Asp Gly
                   1365                1370                1375
   Gln Leu Ala Val Tyr Thr Asn Lys Asp Val Tyr Asp Leu Lys Ala Thr
               1380                1385                1390
   Ile Asn Asp Asn Leu Ser Gly Tyr Ser Leu Gln Val Gly Ser Asp Thr
           1395                1400                1405
   Ala Tyr Gln Asp Lys Thr Tyr Lys Val Phe Asn Glu Asp Phe Phe Lys
       1410                1415                1420
   Asn Arg Asp Ala Val Lys Val Ser Tyr Pro Ile Lys Ala Glu Lys Asp
   1425                1430                1435                1440
   Gly Phe Arg Lys Val Lys Val Thr Leu Thr Asp Gly Ser Asp Asn Lys
                   1445                1450                1455
   Thr Glu Gln Asp Phe Thr Leu Tyr Asn His Gln Ala Asp Leu Glu Ala
               1460                1465                1470
   Pro Glu Val Ser Ala Ser Glu Ser Lys Lys Thr Asn Gln Ala Val Gln
           1475                1480                1485
   Leu Lys Val Gly Asn Leu Ser Asp Val Gln Lys Ser Ala Gly Lys Phe
       1490                1495                1500
   Lys Ala Ala Asp Leu Tyr Tyr Ser Val Asp Gly Lys Ser Trp Thr Lys
   1505                1510                1515                1520
   Leu Asp Lys Asp Thr Val Gln Val Ala Glu Asn Gly Lys Val Glu Phe
                   1525                1530                1535
   Lys Tyr Gln Asp Val Tyr Gly Asn Glu Ser Lys Val Thr Thr Tyr Glu
               1540                1545                1550
   Val Lys Asn Ile Val Lys Glu Val Ala Ala Gln Pro Glu Leu Lys Leu
           1555                1560                1565
   Thr Pro Asp Gly Glu Gly Lys Val Lys Ala Glu Leu Ala Phe Asp Lys
       1570                1575                1580
   Lys Asp Val Asp Lys Asp Phe Asn His Ile Lys Tyr Ser Leu Asp Gly
   1585                1590                1595                1600
   Gly Lys Ser Trp Thr Asp Tyr Lys Asp Ala Phe Thr Leu Thr His Asn
   1605 1610 1615
   Gly Thr Val Glu Phe Lys Ser Tyr Asp Asp Ala Gly Asn Glu Ser Gln
   1620 1625 1630
   Val Tyr Thr Ser Val Val Lys Val Glu Arg Lys Leu Pro Ala Pro Asp
   1635 1640 1645
   Leu Thr Gly Thr Val Glu Ala Asp Lys Ser Val Glu Val Lys Ala Gly
   1650 1655 1660
   Asn Val Glu Gln Ala Glu Lys Asp Ala Asp Gly Lys Val Thr Leu Leu
   1665 1670 1675 1680
   Tyr Ser Thr Asp Gly Lys Asp Trp Ser Lys Val Asp Gly Glu Val Lys
   1685 1690 1695
   Leu Thr Asp Ser Gly Arg Ala Met Phe Lys Tyr Arg Asp Gly Asp Asp
   1700 1705 1710
   Asn Glu Ser Ala Val Val Val Tyr Glu Val Lys Lys Val Val Glu Glu
   1715 1720 1725
   Lys Thr Ala Glu Phe Lys Pro Glu Asn Lys Asp Asp Ser Lys Ser Asp
   1730 1735 1740
   Ser Lys Ser Asp Ser Lys Ser Asp Asn Lys Ala Glu Thr Lys Ser Asp
   1745 1750 1755 1760
   Val Lys Thr Asp Thr Lys Ser Asp Val Lys Ser Asp Gly Asn Gly Glu
   1765 1770 1775
   Arg Lys Ser Asp Asn Lys Pro Ser Ala Lys Asp Asp Lys Lys Thr Asp
   1780 1785 1790
   Ser Lys Thr Thr Ser Lys Ser Ser Ala Lys Lys Thr Ser Ala Lys Lys
   1795 1800 1805
   Asn Lys Lys Ala Ser Lys Lys Ala Ser Lys Lys Thr Val Lys Lys Thr
   1810 1815 1820
   Lys Thr Tyr Lys Lys Val Lys Leu Thr Lys Leu Thr Lys Val Tyr Asn
   1825 1830 1835 1840
   Lys Lys Gly Lys Val Val Gly Lys Leu Lys Lys Lys Thr Ser Val Lys
   1845 1850 1855
   Leu Leu Ser Lys Lys Gln Lys Leu His Gly Lys Tyr Tyr Tyr Arg Val
   1860 1865 1870
   Gly Lys Asn Arg Tyr Ile Leu Ala Ser Ser Leu Pro Lys Lys Thr Lys
   1875 1880 1885
   Lys Val Lys Gln Val Arg Ala Arg Lys Asn Ala Lys Val Tyr Asn Lys
   1890 1895 1900
   Lys Gly Lys Val Val Gly His Leu Lys Lys Lys Gln Lys Val Lys Leu
   1905 1910 1915 1920
   Leu Ser Lys Lys Gln Lys Leu His Gly Lys Tyr Tyr Tyr Arg Ile Gly
   1925 1930 1935
   Lys Asn Arg Tyr Val Asn Ala Asn Val Leu
   1940 1945

## Description

The present invention relates to the proteolytic system of *Lactobacillus bulgaricus*, and especially to the use of the novel recombinant protease PrtP to develop efficient starter strains with accelerated fermentation properties.

### Background of the invention

The proteolytic system is essential to ensure a rapid growth in milk and supplies auxotrophic lactic acid bacteria with amino acids from caseins, the major milk proteins. This system is complex and extensively studied in *lactococci* (Kok et al., FEMS Microbiol. Rev, 87, 15-42, 1990; Smid et al., Appl. Env. Microb., 57, 2447-2452, 1991; Pritchard et al., FEMS Microbiol. Rev., 12, 179-206, 1993).

The first step of milk casein degradation is performed by a cell surface proteinase, PrtP. According to their substrate specificity, two types of PrtPs were distinguished. PI type preferentially cleaves β-casein, and the PIII type degrades α, β and κ caseins. Lactococcal PrtPs are serine protases and show extensive homology with subtilisins secreted by *Bacillus* genus. Lactococcal proteinases are synthetized as inactive preproproteins. A N-terminal signal peptide of 33 residues is removed during crossing through the cytoplasmic membrane and the C- terminus remains anchored in the cell-envelope. Then a maturation process leads to the removal of the pro region (154 residues) and involves a membrane located lipoprotein, PrtM. This 33 κDa protein is encoded by a gene (*prtM*) located immediately upstream and in opposite direction of the prtP gene. Selfdigestion of the carboxy terminus of PrtP results in the release of the enzyme into the culture medium. Incubation of *lactococci* cells in a calcium free buffer stimulates the PrtP selfdigestion and release. *Lactobacilli* have been investigated in a lesser extent, but display a high cell surface proteinase activity with substrate specificity differing from that of *lactococci* (Bosman et al., FEMS Microbial. Rev., 12, p. 72, 1993).

Cell surface proteinases from several *lactobacilli* were purified: *Lb*. *paracasei* subsp. *paracasei* NCDO 151, Lb. *Helveticus* CNRZ 303 and L89, *Lb. bulgaricus* CNRZ 397 (Zevaco et al., Le Lait, 68, 393-408, 1988; Laloi et al., Appl. Microbiol. Biotech., 36, 196-204, 1991; Naes et al., J. Gen. Microbiol., 138, 313-318, 1992; Martin Hernandez et al., Appl. Microbiol. Biotech., 40, 828-834, 1994). The sequence of the gene coding for the proteinase from *Lb. paracasei* was cloned and the deduced amino acid sequence shows 1902 residues and a high homology (96,6%) with lactococcal PrtPs. The presence of a *prtM* gene suggests a maturation process similar to the lactococcal PrtP maturation. In contrast, thermophilic *lactobacilli* proteinases appear different. *Lb. helveticus* CNRZ 303 proteinase is characterized by an original proteolytic specificity towards αₛ₁ casein. The proteinases of *Lb. Bulgaricus* CNRZ397 and *Lb. helveticus* L89 cannot be released from the cell-wall by the procedure using calcium free buffer. Moreover, the *Lb. bulgaricus* enzyme is sensible to serine-and cysteine protease inhibitors.

The cell-wall proteinase of *Lb. bulgaricus* CNRZ397 displays however quite different biochemical properties. This enzyme has been purified from cell-wall extracts obtained after lysozyme treatment and osmotic shock of whole cells. Inhibitors of serine proteases have no effect on this enzyme characterised as a cysteine-protease with a molecular mass of 170 κDa and able to hydrolyse alpha- and beta-caseins. Optimal pH of enzyme activity is 5.5 instead of 7.5 to 8.0 for *Lb. helveticus* proteinase. The level of the cell-wall proteinase synthesis depends on the nature of culture medium: cells grown in a milk medium exhibit a higher activity than cells developed in MRS (rich peptide medium).

In Mol. Gen Genet. 248 (1995), 407 - 416 an IS-element of *Lactobacillus delbrueckii* subsp. *bulgaricus* is described, which has been found to be located between two gene loci, namely that of the LacZ gene and the prtP gene. In addition, a restriction map of said area of the bacterial genome indicating the approximate or supposed loci of the lacS and the lacZ gene, the ISL3 element and the prtP-gene is disclosed.

In J. of Appl. Bacteriol. 41 (1976), 175-184 a protease activity during growth of *Lactobacillus bulgaricus* is described. The authors of this paper report that the protease activity found - measured by means of one specific substrate (dimethylcasein) - is bound to the cell membrane of the micro-organism.

The present invention has the objective to use an effective gene of a protease to develop dairy starter strains with accelerated fermentation properties. This protease should be active without a further maturation step involving another protein. Moreover, the level of activity of this protease should be sufficient, but not too high, to permit an accelerated fermentation of dairy products, without accelerating the degradation of these products at chilled temperature during storage. Finally, this protease should produce amino acid and peptides precursors involved in the formation of typical dairy flavours.

### Summary of the invention

The invention relates to a recombinant cell of a dairy starter strain expressing a recombinant protease prtP of Lactobacillus bulgaricus having the amino acid sequence SEQ m No: 2 or a functional derivative thereof, which derivative has an amino acid sequence at least 50 % identical to the sequence of SEQ Yid. NO. 2.

The invention further relates to a method for producing the recombinant protease PrtP comprising, cultivating recombinant cells according to the invention in a suitable growth medium under conditions that the cells express the said recombinant protease, and optionally isolating the said recombinant protease in the form of a contentrate.

In a further aspect of the invention, the use of these recombinant cells is provided for the manufactureof fermented dairy products.

### Short description of the drawings

Figure 1: fermentation of a milk by *Lactococcus lactis* recombinant strains expressing the protease PrtP (see example 2: transformants with plasmid pLL82).
Figure 2: fermentation of a milk by *Lactococcus lactis* recombinant strains containing but not expressing the protease PrtP (see example 2: plasmid pLL81).
Figure 3: restriction map of the *prtP* region.

### Detailed description of the invention

For purpose of this disclosure and claims, the term *"prtP"* refers to the gene encoding the related protease "PrtP" of *Lactobacillus bulgaricus*.

Within the context of the present invention the expression "functional derivative" may comprise all amino acid sequences which differ by substitution, deletion, addition of some amino acids but which keep their original activities or functions. A protein may be generally considered as a derivative to another protein, if its sequence is at least 50% identical to the protein, preferably at least 70%, in particular 90%. In the present disclosure, the identity is determined by the ratio between the number of amino acids of a derivative sequence which are identical to those of PrtP and the total number of or amino acids of the said derivative sequence.

Likewise, the expression "a DNA encoding the protease PrtP" may comprise any DNA encoding the same or a derivative protein due to the degeneracy of the genetic code or to the cross species variation. A DNA molecule may be generally considered as a derivative to another DNA molecule, if its sequence is at least 40% identical to the another DNA molecule, preferably at least 60%, in particular 80%. In the present disclosure, the identity is determined by the ratio between the number of bases of a derivative sequence which are identical to those of nucleotides 794 to 6631 from SEQ ID NO:1 and the total number of bases of the said derivative sequence.

The DNA molecule encoding the prtP protease may be obtained in substantially purified form, by using the method described in the following examples, from any strain of *Lactobacillus bulgaricus*, more particularly from the strain *L. bulgaricus NCDO1489* (NCDO comes from the former name "National Collection of Dairy Organisms" of the NCFB - National Collection of Food Bacteria - which in an International Depository Authority under the Budapest Treaty; see NCFB catalogue of strains, 1986, p 85, No 1489).

Alternatively, the DNA molecule may be recovered also from other genera or species of bacteria by use of DNA probes derived from a DNA sequence of the invention in a stringent hybridisation assay.

In a further aspect, the DNA molecule may also be synthesised from sequences given in the sequence listing below, multiplied *in vitro* for instance by using the polymerase chain reaction or multiplied *in vivo* for instance in bacteria of the species *Escherichia coli*, *Lactococcus lactis*, or *Streptococcus thermophilus*.

The DNA molecule comprises at least the DNA sequence SEQ ID NO:1 in particular from nucleotide 794 to 6631 of SEQ ID NO:1 encoding the prtP gene.

The DNA molecule may also comprise a sequence which is a derivative (see definition) to.the above sequences.

The DNA molecule can be present in a vector, such as a replicative plasmid or an integrative circular or linearized non replicative plasmid,. for example.

The DNA molecule can also comprise, operably linked to the said DNA, regulatory sequences native to the organism from which derives the nucleotide sequence. The said native regulatory sequences can be a promoter, a terminator, a Shine-Dalgarno sequence, an enhancer, or a sequence encoding a leader peptide, for example, that regulate expression of *prtP*.

In another embodiment, regulatory sequences can be native sequences that regulate a different gene in the said organism of origin or that regulate a different gene in a foreign organism, for example. A regulatory sequence other than the native regulatory sequence will generally be selected for its high efficiency. It is also possible to select as regulatory sequence a promoter on the basis of other desirable characteristics, for example thermo inducibility, or a sequence encoding a peptide signal which will permit excretion of the protein.

If heterologous expression is preferred, meaning that the prtP genes are expressed in another organism than the original host (strain, variety, species, genus, family, order, class or division) the regulatory sequence is preferably derived from an organism similar or equal to the expression host. For example, if the host is a yeast cell, then the regulatory sequence will be derived from a yeast cell. The promoter suitable for constitutive expression, preferably in a fungal host, may be a promoter from the following genes: glycerolaldhehyde-3-phosphate dehydrogenase, phospho-glycerate kinase, triose phosphate isomerase and acetamidase, for example. Promoter suitable for inducible expression, preferably in a fungal host, may be a promoter from the following genes: endoxylanase IIA, glucoamylase A, cellobiosehydrolase, amylase, invertase, alcohol dehydrogenase and amyloglucosidase. The selection of a desirable regulatory sequence operably linked to a sequence of the invention and capable of directing the expression of the said nucleotide sequence is considered to be obvious to one skilled in the art.

The DNA molecule may also comprise a selection marker to discriminate host cells into which the recombinant DNA material has been introduced from cells that do not comprise the said recombinant material. It may also comprise at least one suitable replication origin. Suitable transformation methods and suitable expression vectors provided with a suitable transcription promoter, suitable transcription termination signals and suitable marker genes for selecting transformed cells are already known in the literature for many organisms including different bacteria, fungal and plant species.

Overexpression of proteins may be achieved by incorporation of the DNA molecule in an expression host, the said DNA molecule comprising one or more regulatory sequences which serve to increase expression levels of the protein(s). The overexpression can be further achieved by introducing a multicopy of the DNA molecule, for example.

The invention encompasses a recombinant cell of a dairy starter strain comprising the DNA molecule described above. These cells may be derived from the group of fungal cells in particular of the genus *Aspergillus*, yeast cells in particular of the genera *Saccharomyces, Kluyveromyces, Hansenula* and *Pichia*, bacterial cells in particular Gram-positive bacteria of the genera *Bacillus*, *Lactobacillus*, *Streptococcus* in particular *Streptococcus thermophilus, Bifidobacteria*, *Staphylococcus* and *Lactococcus* in particular *Lactococcus lactis*, and plant cells in particular of the tree and vegetable groups, for example.

Recombinant cells may comprise the DNA molecule described above stably integrated into the chromosome or on a replicative plasmid. Preferably, the DNA molecule is integrated into the chromosome by using the process described in EP564966, i.e.,
(1) transforming a host strain microorganism with a donor plasmid which does not replicate in the host strain, wherein the donor plasmid comprises a vector backbone and a sequence comprising a promoterless foreign gene (*prtP* or similar genes) operably integrated into at least a part of an operon of the host strain, maintaining the frame and the function of the genomic operon of the host strain;
(2) identifying cointegrate transformants in which the complete donor plasmid is integrated into the genomic operon of the host strain;
and (3) selecting an integrant transformant from the cointegrate transformants, wherein the genome of the selected integrant transformant does not include the vector backbone of the donor plasmid but does include the foreign gene, which is operably integrated into the conserved genomic operon and which is stably maintained and expressed due to selective pressure on the correct functioning of the essential cistron upon growth in a standard medium.

Progeny of an expression host comprising a DNA molecule as described is also included in the present invention. Accordingly, the invention is directed to a cell comprising a recombinant DNA molecule of the prtP gene above, in any of the embodiments described above, wherein the said cell is able to integrate the PrtP protease or functional derivatives into the cell wall or the cell membrane or secrete the enzymes into the periplasmic space or the culture medium. The secreting route to be followed by recombinant proteins according to the invention will depend on the selected host cell and the composition of the recombinant DNA according to the invention. Most preferably, however, the protein will be bound to the outer cell-wall. To this end, the cell according to the invention can comprise recombinant DNA further comprising operably linked DNA encoding foreign leader sequences (pre or prepro), for example.

The invention is also directed to a process for producing recombinant protein(s) comprising, providing recombinant cells according to the invention in a suitable growth medium under conditions that the cells express the said recombinant protein(s), and optionally isolating the said recombinant protein(s) in the form of a concentrate. The selection of the appropriate medium may be based on the choice of expression host and/or based on the regulatory requirements of the DNA recombinant material. Such media are well-known to those skilled in the art.

After fermentation, the cells can be removed from the fermentation broth by centrifugation or filtration. Depending on whether the host cells have secreted the recombinant protein(s) into the medium or whether the said protein(s) are still connected to the host cells in some way either in the cytoplasm, in the periplasmic space or attached to or in the membrane or cell wall, the cells can undergo further treatment to obtain the recombinant protein(s). In the latter case, where the recombinant protein(s) are still connected to the cells, recovery may be accomplished by rupturing the cells for example by high pressure, sonication, enzymatic digestion or simply by cell autolysis followed by subsequent isolation of the desired product. In this context, the method as described by Laloi et al. in Appl. Microb. Biotech., 36 , 196-204, 1991 may be applied. The protein(s) can be separated from the cell mass by various methods, such as ultrafiltration, and then subsequently precipitated with an organic solvent. The isolated protein(s) may be further purified by conventional methods such as precipitation and/or chromatography.

The invention is also directed to the use of recombinant cells according to the invention, for the manufacture of fermented dairy products, in particular yoghurt, acidified milk and cheese.

Indeed, of all processes involved in the ripening from cheese, for example Cheddar and Gouda, the degradation of milk proteins by proteinases from starter cultures is the most important process. Ideally, the objective in accelerating ripening should be to accelerate all the desirable reactions involved in ripening in a balanced way while controlling the undesirable ones. However, since the key reactions responsible for the unique flavour are not known in most cases, a more or less empirical approach has to be adopted. Since the glycolytic reactions occur very quickly and since the modification of lactose in most cheeses is not the fermentation rate limiting step, there is apparently no need to accelerate glycolysis and subsequent reactions. Likewise, lipolysis is not important, is in fact undesirable, in most varieties. Therefore, attempts to accelerate ripening have concentrated on proteolysis. Essentially, there are two approaches to accelerate cheese ripening. Although elevated temperatures may give satisfactory results with a 50% reduction in ripening time, industry appears to be reticent to apply this technique. Development of engineered "super" starters, which may accelerate ripening are therefore awaited.

On the other hand, although yoghurt starter cultures are considered to be only weakly proteolytic, *S. thermophilus* and *L. bulgaricus* may, during the fermentation, cause a significant degree of proteolysis, and this activity may be important for the following reasons:
(a) The enzymatic hydrolysis of milk proteins results in the liberation of peptides of varying sizes and free amino acids, and these possible changes can affect the physical structure of yoghurt.
(b) The liberation of amino acids into the milk is essential for the growth of *S. thermophilus*. Indeed *S. thermophilus* does not possess substantial extracellular proteolytic activity and the amino acid and free peptide content of milk is not high enough to promote its full growth.
(c) Although amino acids and peptides may not contribute directly to the flavour of yoghurt, they do act as precursors for the multitude of reactions which produce flavour compounds.

Therefore, there is a need to develop dairy starter strains with accelerated fermentation properties, or capable to full grow in a milk (*S. thermophilus* for instance).

However, these starters should not exhibit a too high protease activity, otherwise the advantage of the accelerated ripening or fermentation is counter-balanced by an accelerated degradation of the product. Therefore, the protease activity should be carefully chosen, so that at chilled temperature it does not degrade the product.

It has been surprisingly observed, that the recombinant protease PrtP according to the invention fulfils these needs.

In order to improve the shelf stability of dairy products fermented by cells according to the invention, it is possible to make dependent the gene encoding of the said PrtP to a temperature or pH sensitive promotor selected to be inhibited at chilled temperatures, that is to say 9°C to 12°C, or at acid pH, for example lower than 5.5. Such promotors are already known to the skilled person.

Alternatively, the gene itself may be mutated and rendered sensitive to temperature or pH. Therefore, it is possible to use a recombinant cell according to the invention, said cell expressing a recombinant derivative of the protease PrtP according to the invention, said derivative exhibiting decreased activity at a rate of at least 20% less than the protease having the amino-acid sequence SEQ ID NO:2, under the storage conditions of the fermented dairy product, but where the said derivative retains at least 90% of its activity at the production conditions of the fermented dairy product when compared to the protease having the amino-acid sequence SEQ ID NO:2.

Preferably, the derivative protease according to the invention exhibits decreased activity at a temperature below 20°C, or at a pH below 5.5.

Such variants and recombinant cells are readily obtainable by use of a process comprising:
a) inserting the *prtP* gene in a vector;
b) performing mutations on a plurality of vectors of step a);
c) selecting those vectors of step b) in which said gene is mutated so that when a selected vector is used to transform an organism, expression of the selected gene produces a variant enzyme which exhibits decreased activity at a rate of at least 20% less than the PrtP enzyme having the amino acid sequence SEQ ID NO:2., under the storage conditions of the fermented dairy product, but where the said derivative retains at least 90% of its activity at the production conditions of the fermented dairy product when compared with the PrtP enzyme having the amino acid sequence SEQ ID NO:2.
d) inserting and expressing a selected gene coding for the variant enzyme of step c) into an organism suitable for the production of the fermented food product.

This method has already been used for the development of β-galactosidase variants, as described in EP 402 450.

The present invention is further illustrated hereafter, and not limited, by a supplemental description which refers examples of characterisation of DNA molecules, cells, proteins and use according to the invention, in which all parts, ratios, and percentages are expressed on a weight basis unless otherwise stated, with reference to the accompanying drawings.

Methods involving DNA techniques were essentially performed as described in the Book of Sambrook et al. (Sambrook et al., Molecular Cloning, A Laboratory Manual, Cold Spring Harbor Laboratory Press, U.S.A., 1989).

### Example 1:

### DNA sequencing of the prtP gene

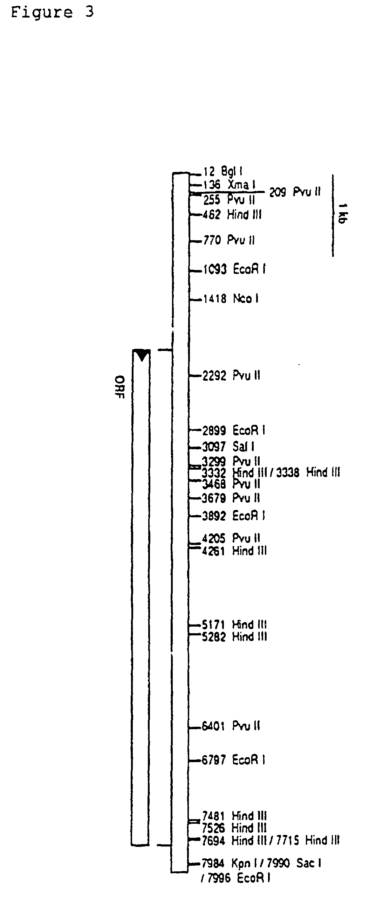

From the *lacZ* gene of the *L. bulgaricus* NCDO 1489 strain, reverse PCR allowed us to pick up the DNA region downstream to the *lacZ* gene. (See figure 3). The PCR fragment was cloned in pUC19 and sequenced (Yanisch et al., Gene, 33, 103-119, 1991). Search for protein homology in protein libraries revealed an identity of 27.8% between the deduced amino acid sequence with the PrtP from *Lb. paracasei* NCDO151. Then, the region downstream *lacZ* and including the *prtP*-like gene was determined in a cascade PCR strategy using synthetic primers as the known sequence progressed. A restriction map of the *prtP* gene location from *L. bulgaricus* is presented in Fig. 3.

The nucleotide sequence of the *prtP* gene is presented in the sequence listing below (see nucleotides 794 to 6631 of SEQ ID NO:1).

The *prtP* gene is under the control of a promoter located from nucleotide around 588 to 793 of SEQ ID NO:1.

The open reading frame (ORF: nucleotide 794-6631) displays homology with lactococal *prtP* genes. The first encoding DNA codon (ATG) is preceded by a Shine-Dalgarno sequence matching well the 16S rRNA from *lactobacilli* species (see nucleotide 784 to 790 of SEQ ID NO:1). Moreover, the N-terminus amino acid sequence translated looks like a signal sequence of PrtPs. The upstream contains three characteristic boxes and might be the promoter region (see nucleotide 632 to 659 of SEQ ID NO:1). Moreover, 5 nucleotides upstream -35 box, there is a third recognition signal which is called UP-element and characterised by oligo A and T stretches (see nucleotide 608-627 of SEQ ID NO:1). The UP element has already been found in lactococcal *prtP* and *Lb. delbrueckii* subsp. *lactis* promoters and is known to enhance transcription (Kok et al., Appl. Environ. Microbiol., 54, 231-238, 1988).

The coding sequence of *prtP* gene is 5,841 nucleotides long which is in the range of length of *ptrP* genes. The GC content of *prtP* gene is estimated at 47.6% which is very similar to the ratio described for the *prtP* genes of *Lb. paracasei* and *Lc. lactis* NCDO 763 (47.5 and 47.2%, respectively). It is interesting to stress that *Lb. bulgaricus* genome exhibits a GC content of 54% and *Lc. lactis* of 38% (Kilpper-Balz et al., Cur. Microbiol., 7, 245-250, 1982). So, *prtP* gene significantly differs from other *L. bulgaricus* genes (*lacZ*, *lacS* and *pepIP*) which show a CG content of 52.5, 53.1 and 56.9%, respectively (Leong et al., J. Bacteriol., 173, 1951-1957, 1991, Atlan et al., Microbiol., 140, 527-535, 1994).

The expected proteinase PrtP consists of 1,946 amino acids and is characterised by a predicted Mr of 212,271.

The amino acid sequence of this PrtP protease is presented in the listing sequence below (see SEQ ID NO:2). This length fits in well with other described PrtPs: 1,902 residues for *Lb. paracasei, Lc. cremoris* Wg2 and *Lc. lactis* NCDO 763, and 1,962 residues *for Lc. cremoris* SK11 associated with a duplication of 60 amino acids at the COOH extremity.

*L. bulgaricus* PrtP shows 27% of identity with *Lb. paracasei* PrtP over the first 1,806 residues and up to 39.5% when only the first 820 residues were compared. The C-terminus part of the cell surface proteinase did not share any homology with a PrtP proteinase.

The major amino acid of PrtP is lysine (12%) instead of leucine for other *Lb.bulgaricus* enzymes (LacS, Beta-galactosidase and PepIP) or threonine for other lactococcal and *Lb. paracasei* PrtPs. Codon usage was investigated and is similar to other *L. bulgaricus* enzymes (LacS, β-galactosidase and PepIP) for lysine and glycine; but serine, aspartic acid, and valine are preferentially coded as *lactococci*. Finally, alanine codon preference is specific for PrtP.

### PrtP and cell envelope location

At the amino acid level, the homology of sequences between N-terminus regions of PrtP exhibits a very positively charged N-extremity followed by an α-helix with a high content in leucine and alanine. This domain closely resembles signal peptides of exported proteins of Gram positive bacteria. The prepro portion of the prtP enzyme is thus located from amino-acid 1 to 192 of SEQ ID NO:2.

The absence of a prtM-like gene located immediately upstream or downstream the *prtP* gene and expressing a functional chaperone gives rise to two hypotheses. The maturation and export of PrtP could be processed by a PrtM-like chaperone encoded by a gene located anywhere on the chromosome; however, it cannot be ruled out that a general chaperone acts on different extracytoplasmic proteins, PrtP included. On the other hand, the PrtP may preferably not be processed by a PrtM-like chaperone, since the recombinant PrtP is functional in different host system (see examples 2 and 3).

In contrast to the proteinases PrtPs, PrtP from *L. bulgaricus* is never recovered in the culture medium and, consequently, is devoid of the ability of removal of the C-extremity by selfdigestion. A portion of the prtP enzyme is thus believed to be an anchorage sequence which permits to the enzyme to be stably maintained in the outer membrane of the cell. This portion is thus located from amino-acid 1883 to at least amino-acid 1915 of SEQ ID NO:2.

Once the first step of casein catabolism, the peptides released by the PrtP action are different from those resulting from the hydrolysis by PrtPs. Other steps of the proteolytic system of *Lb. bulgaricus* are suspected of being different. For instance, Lb. bulgaricus exhibits high activities towards substrates containing proline (Sasaki et al., FEMS Microbiol. Rev. 12, p 75D16, 1993).

### Example 2

*L. bulgaricus* NCDO 1489 DNA was purified by the spooling method (Delley et al., Appl. Environ. Microbiol., 56, 1967-1970, 1990). Cell wall associated protease gene (*prtP*) was amplified by PCR using the polymerases Biotag (bioprobe, FR) and a 1/20 dilution of Pfu (cloned Pfu DNA polymerase Stratagene, USA) and the conditions 92°C for 1mn, 55°C for 2 mn and 72°C for 6mn (Saiki et al., Science, 239, 487-491, 1988). To this end, the *prtP* gene was amplified without its promotor (primers having respectively for sequence the nucleotides 763-787 and 6831-6858 of SEQ ID NO:1) and with its promotor (primers having respectively for sequence the nucleotides 557-583 and 6831-6858 of SEQ ID NO:1). PCR products were cleaved with BamHI and XbaI and ligated in XbaI/BglII digested pNZ124 (Platteeuw et al., Applied and Env. Microbio., 60, 587-593,1994)

Recombinant plasmids pLL81 (promoter free *prtP*) and pLL82 (*prtP* with promoter) were introduced in *Lactococcus lactis* MG1363 (plasmid free) by electroporation (Holo and Nes, Applied and Env. Microb., 55, 3119-3123, 1989).

Transformants were grown in M17 with 1% glucose (Difco laboratory) in the presence of 5 µg/ml chloramphenicol. As an example, a transformant comprising the plasmid pLL82a (the *prtP* gene with its promoter) has been deposited at the Pasteur Institute, 28 rue du Dr. Roux, F-75724 Paris 15, France, where it received, on May 24, 1996, the deposit number CNCM I-1713.

Transformants were precultured in 10% reconstituted skim-milk containing 0.1% yeast extract, 1% glucose and 5 µg/ml chloramphenicol. Growth was followed in pasteurised (80°C, 30mn) 10% reconstituted skim-milk containing 1% glucose and 5 µg/ml chloramphenicol by impedence measurement (RABIT: rapid automated bacterial impedence technique, Don Whitley scientific limited, England).

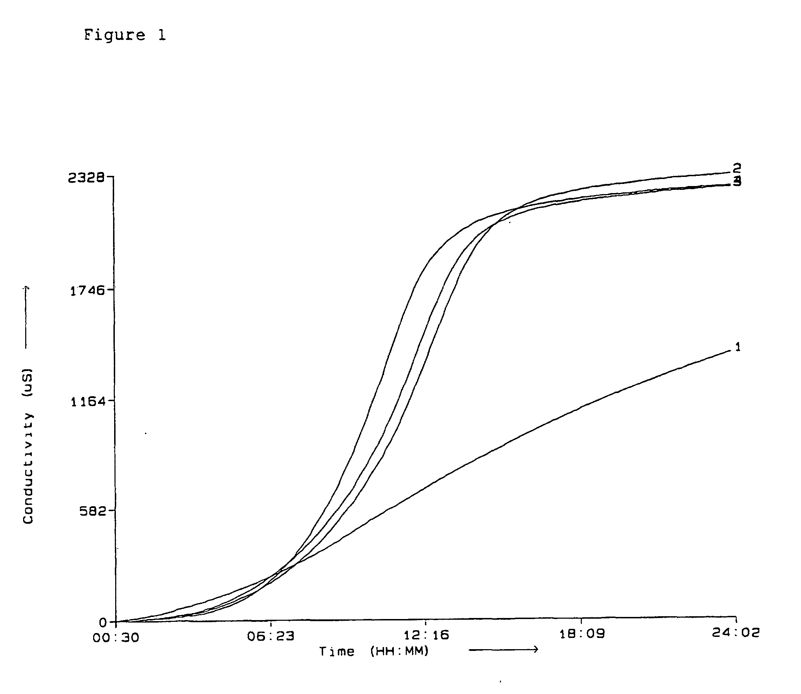

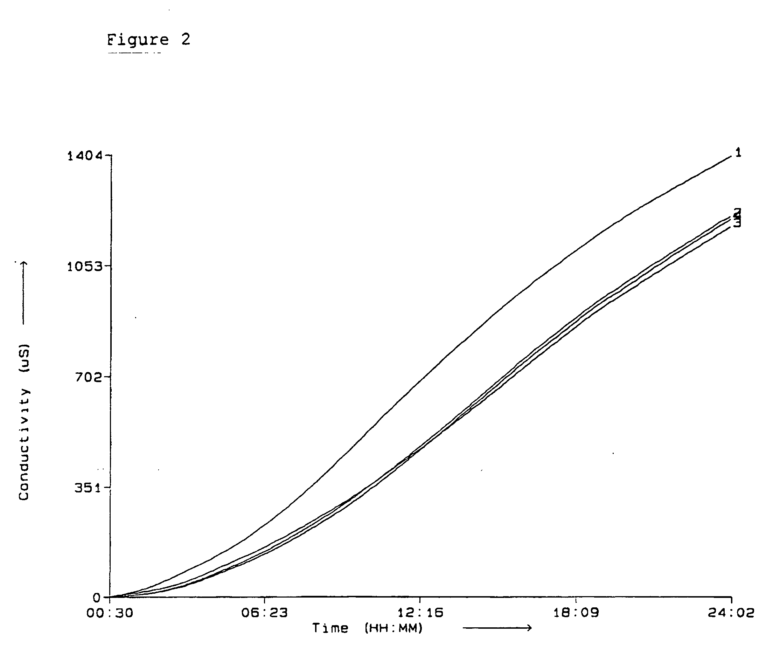

Figure 1 presents the fermentation results: plot 1 designates the *Lactococcus lactis* MG1363 strain without plasmid, plots 2 to 4 designate 3 independent *Lactococcus lactis* transformants comprising the plasmid pLL82 (*prtP* with promotor). As a control Figure 2 shows fermentation results with *Lactococcus lactis* transformants containing plasmid pLL81 (*prtP* without promoter): plot 1 designates the *Lactococcus* lactis MG1363 strain without plasmid, plots 2 to 4 designate 3 independent *Lactococcus lactis* transformants comprising the plasmid pLL81.

### Example 3

Plasmids pLL81 and pLL82 of Example 2 were used to transform a *Streptococcus thermophilus*. The transformants were precultured and cultured in the same manner as described in Example 2. The results are similar to those presented in figures 1 and 2.

### Example 4

A frozen mixed starter comprising a mixture of a strain *Streptococcus thermophilus* containing an expressed *Lb. bulgaricus prtP* gene (see example 3) and the strain *Lactobacillus delbruckii subsp. bulgaricus* CNCM I-1420 is prepared as follows. To this end, a skimmed milk is reconstituted using 10% of skimmed milk powder, 1% of yeast extract is added, and the mixture is sterilized to 115°C for 30 minutes and then cooled to a temperature of approximately 42°C. To this milk there is added 1% of a fresh preculture of the strain *S. thermophilus* and 2% of a fresh preculture of the strain *L. delbruckii subsp. bulgaricus*. The milk is subsequently incubated at a temperature of approximately 42°C until a pH of 4.7 is reached. The milk is cooled to a temperature of 4°C. Then 5% of sterile glycerol is added and the mixture is frozen at -75°C.

Stirred yoghurts are then prepared by direct inoculation with this frozen starter. To this end, the milk is prepared from full-cream milk comprising 3.7% of fat and 2.5% of skimmed milk powder. 40 1 of this milk is sterilised at 105°C for 2 minutes, subsequently homogenised at 75°C and 300 bar (first stage) and finally cooled to a temperature of approximately 43°C. This milk is then fermented with 10ml of the frozen starter at 43°C until a pH of approximately 4.65 is reached and then it is cooled to 4°C. The results show that we gain time during the fermentation, in comparison with traditional starters. Moreover, the yoghurt is stable during several weeks at 4°C.

### Example 5

An acidified milk is prepared traditionally with the same *S. thermophilus* strain expressing the *Lb. bulgaricus prtP* gene (see example 3). The results show that the milk can be acidified up to a pH below 4.9. Moreover, the acidified milk is stable during several weeks at 4°C.

### Example 6

*Lactococcus lactis* CNCM I-1713 is used in a conventional process for making Cheddar cheese. To a milk is added 0.8% of a fully ripened bulk starter containing a high population of a commercial *Leuconostoc mesenteroides* subsp. *cremoris* and the strain *Lactococcus lactis* CNCM I-1713, and 5.10⁶ CFU/ml of a commercial dried *Lactobacillus plantarum*. The mixture is allowed to ripen for 1 hour after which single-strength calf rennet is added. The proper coagulum is formed within 30 minutes at which point cutting occured. Standard cheesemaking procedures are followed to provide a cheddared curd which are milled when a pH of 5.4 is reached. The milled curd is salted to provide a finished product containing 1.8% salt. The finished hooped curd has a final moisture content of 37% and fat content of 33%. The blocks are then shrink film wrapped and aged at 10°C for 3 months which results in the production of a typical aged (6 months) cheddar cheese flavour. Longer aging at 10°C results in strongly aged cheddar cheese flavors which simulate 12 month flavors with only 6 month storage. The flavor development rate can be substantially reduced by lowering the aging temperature from 10°C to 7°C or below.

## Claims

1. Recombinant cell of a dairy starter strain, said cell being selected from the group consisting of *Lactococci* and *Streptococci*, **characterized in that** a nucleotide sequence has been introduced in said cell, encoding the protease PrtP of *Lactobacillus bulgaricus* having the amino acid sequence SEQ ID NO: 2 or a functional derivative of said protease PrtP having a sequence being at least 90 % identical to said nucleotide sequence, and **in that** said cell is expressing said protease PrtP of *Lactobacillus bulgaricus* or said functional derivative thereof.

2. The recombinant cell of a dairy starter strain according to claim 1, wherein the gene encoding said recombinant protease PrtP or said functional derivative thereof is under the control of a promoter.

3. The recombinant cell of a dairy starter strain according to claim 2, wherein said promoter is a *Lactobacillus bulgaricus* promoter of the prtP gene.

4. The recombinant cell of a dairy starter strain according to claim 3, wherein said promoter is the *Lactobacillus bulgaricus* promoter of the prtP gene, having the nucleotide sequence from nucleotide 588 to 793 of SEQ ID NO: 1.

5. The recombinant cell of a dairy starter strain according to any of the preceding claims, wherein said cell is selected in the group consisting of *Lactococcus lactis* and *Streptococus thermophilus*.

6. The recombinant cell of a dairy starter strain according to any of the preceding claims, wherein said cell is *Lactococcus lactis* CNCM I-1713.

7. A method for producing a recombinant protease PrtP of *Lactobacillus bulgaricus*, said method comprising: cultivating recombinant cells expressing a recombinant protease PrtP according to any of the preceding claims in a suitable growth medium under condition that the cells express said recombinant protease.

8. Use of a recombinant cell of a dairy starter strain according to claim 1 to 6, in the manufacture of fermented dairy products.

9. Use of a recombinant cell of a dairy starter strain according to claim 8, wherein said dairy products are selected from the group consisting of yoghurt, acidified milk and cheese.

10. Use of a recombinant cell of a dairy starter strain according to claim 8, said cell expressing a recombinant DNA encoding said protease PrtP having the amino acid sequence SEQ ID NO: 2, under the dependence of a temperature or pH sensitive promoter.

## Patentansprüche

1. Rekombinante Zelle eines Molkerei-Starter-Stammes, wobei die Zelle ausgewählt ist aus der Gruppe bestehend aus *Lactococci* und *Streptococci*, **dadurch gekennzeichnet, dass** eine Nukleotidsequenz in die Zelle eingeführt wurde, die für die Protease PrtP von *Lactobacillus bulgaricus* codiert, die die Aminosäuresequenz SEQ ID Nr.2 aufweist, oder für einen funktionalen Abkömmling der Protease PrtP, der eine Sequenz mit mindestens 90% Identität zu der Nukleotidsequenz aufweist, und dass die Zelle die Protease PrtP von *Lactobacillus bulgaricus* oder den funktionalen Abkömmling davon exprimiert.

2. Rekombinante Zelle eines Molkerei-Starter-Stammes nach Anspruch 1, worin das Gen, das für die rekombinante Protease PrtP oder den funktionalen Abkömmling davon codiert, unter der Kontrolle eines Promotors steht.

3. Rekombinante Zelle eines Molkerei-Starter-Stammes nach Anspruch 2, worin der Promotor ein *Lactobacillus bulgaricus* Promotor des prtP Gens ist.

4. Rekombinante Zelle eines Molkerei-Starter-Stammes nach Anspruch 3, worin der Promotor der *Lactobacillus bulgaricus* Promotor des prtP Gens ist, der die Nukleotidsequenz von Nukleotid 588 bis 793 von SEQ ID Nr. 1 aufweist.

5. Rekombinante Zelle eines Molkerei-Starter-Stammes nach einem der vorstehenden Ansprüche, worin die Zelle ausgewählt ist aus der Gruppe bestehend aus *Lactococcus lactis* und *Streptococcus thermophilus*.

6. Rekombinante Zelle eines Molkerei-Starter-Stammes nach einem der vorstehenden Ansprüche, worin die Zelle *Lactococcus lactis* CNCM 1-1713 ist.

7. Verfahren zur Herstellung einer rekombinanten Protease PrtP von *Lactobacillus* *bulgaricus*, wobei das Verfahren umfasst: Züchten rekombinanter Zellen, die eine rekombinante Protease PrtP gemäß einem der vorstehenden Ansprüche exprimieren, in einem geeigneten Wachstumsmedium, unter der Bedingung dass die Zellen die rekombinante Protease exprimieren.

8. Verwendung einer rekombinanten Zelle eines Molkerei-Starter-Stammes nach Anspruch 1 bis 6 in der Herstellung von fermentierten Molkereiprodukten.

9. Verwendung einer rekombinanten Zelle eines Molkerei-Starter-Stammes nach Anspruch 8, wobei die Molkereiprodukte ausgewählt sind aus der Gruppe bestehend aus Yoghurt, gesäuerter Milch und Käse.

10. Verwendung einer rekombinanten Zelle eines Molkerei-Starter-Stammes nach Anspruch 8, wobei die Zelle eine rekombinante DNA, die für die Protease PrtP codiert, die die Aminosäuresequenz SEQ ID Nr. 2 aufweist, in Abhängigkeit eines Temperatur- oder pHempfindlichen Promotors exprimiert.

## Revendications

1. Cellule recombinante d'une souche de démarrage laitière, ladite cellule étant sélectionnée à partir du groupe consistant en *Lactococci* et *Streptococci*, **caractérisée en ce qu'**une séquence de nucléotides codant la protéase PrtP de *Lactobacillus bulgaricus* ayant une séquence d'acides aminés SEQ ID NO :2 ou un dérivé fonctionnel de ladite protéase PrtP ayant une séquence qui est identique à au moins 90 % à ladite séquence de nucléotides, a été introduite dans ladite cellule et **en ce que** ladite cellule exprime ladite protéase de *Lactobacillus bulgaricus* ou le dérivé fonctionnel de celle-ci.

2. La cellule recombinante d'une souche de démarrage laitière selon la revendication 1 dans laquelle le gène codant ladite protéase recombinante PrtP ou ledit dérivé fonctionnel de celle-ci est sous le contrôle d'un promoteur.

3. La cellule recombinante d'une souche de démarrage laitière selon la revendication 2 dans laquelle le promoteur est un promoteur *Lactobacillus bulgaricus* du gène prtP .

4. La cellule recombinante d'une souche de démarrage laitière selon la revendication 3 dans laquelle le promoteur est un promoteur *Lactobacillus bulgaricus* du gène prtP ayant la séquence de nucléotides allant du nucléotide 588 à 793 de SEQ ID NO :1.

5. La cellule recombinante d'une souche de démarrage laitière selon l'une quelconque des revendications précédentes dans laquelle ladite cellule est sélectionnée dans le groupe consistant en *Lactococcus lactis* et *Streptococcus thermophilus*.

6. La cellule recombinante d'une souche de démarrage laitière selon l'une quelconque des revendications précédentes dans laquelle ladite cellule est *Lactococcus lactis* CNCM I-1713.

7. Une méthode pour produire une protéase recombinante PrtP de *Lactobacillus bulgaricus*, ladite méthode comprenant : cultiver des cellules recombinantes exprimant une protéase PrtP recombinante selon l'une quelconque des revendications précédentes dans un milieu de culture approprié, dans des conditions telles que les cellules expriment ladite protéase recombinante.

8. Utilisation d'une cellule recombinante d'une souche de démarrage laitière selon les revendications 1 à 6 dans la fabrication de produits laitiers fermentés.

9. Utilisation d'une cellule recombinante d'une souche de démarrage laitière selon la revendication 8 dans laquelle lesdits produits laitiers sont sélectionnés à partir du groupe consistant en yogourt, lait acidifié et fromage.

10. Utilisation d'une cellule recombinante d'une souche de démarrage laitière selon la revendication 8, ladite cellule exprimant un ADN recombinant codant ladite protéase ayant une séquence d'acides aminés SEQ ID NO :2 sous la dépendance d'un promoteur sensible à la température ou au pH.
